# EUROPÄISCHE PATENTSCHRIFT

(11) **EP 1 191 381 B1**
(45) Veröffentlichungstag und Bekanntmachung des Hinweises auf die Patenterteilung: **08.08.2007**
(21) Anmeldenummer: 01122891.3
(22) Anmeldetag: 24.09.2001
(51) Int. Cl.: G02B 21/22, G02B 21/00, A61B 3/13

(54) **Stereo-Mikroskopieanordnung**
Stereoscopic microscopy device
Dispositif stéréoscopique pour microscopie

(30) Priorität: 26.09.2000 DE 10047617
(43) Veröffentlichungstag der Anmeldung: 27.03.2002
(60) Teilanmeldung: 02027423.9
(73) Patentinhaber: Carl Zeiss Surgical GmbH, 73447 Oberkochen (DE)
(72) Erfinder: Strähle, Fritz, Dr., 73540 Heubach (DE)
(74) Vertreter: Schorr, Frank Jürgen

(56) Entgegenhaltungen:
- ES-A- 2 116 933
- US-A- 4 710 000
- US-A- 4 991 947
- US-A- 5 009 487
- US-A- 5 200 773
- US-A- 5 535 060

## Beschreibung

Die vorliegende Erfindung betrifft eine Stereo-Mikroskopieanordnung zur Betrachtung eines Objekts oder eines von einem Objekt erzeugten Zwischenbilds. Hierbei kann das Zwischenbild insbesondere ein höhen- und seitenverkehrtes Abbild eines Objekts sein, und die Mikroskopieanordnung kann zum Einsatz in der Mikrochirurgie, insbesondere der Augenchirurgie, vorgesehen sein.

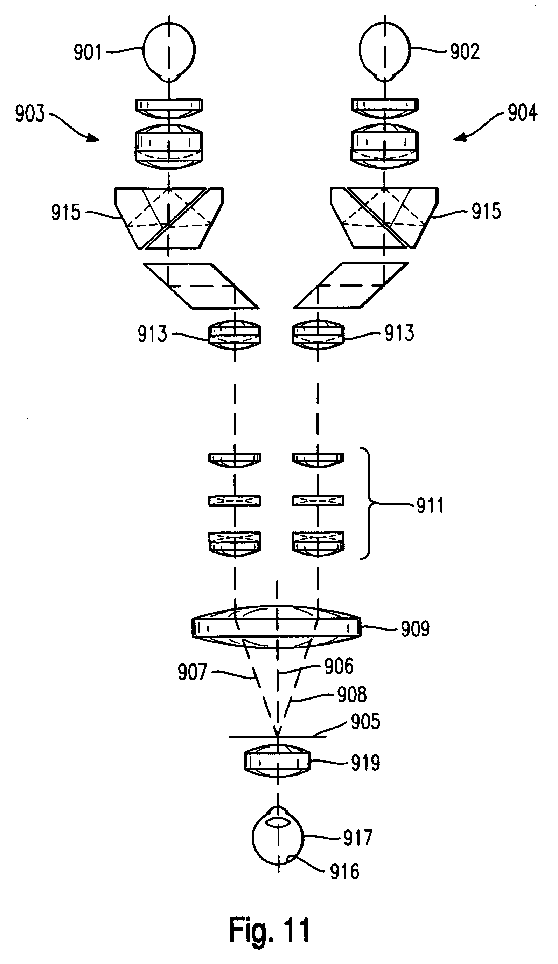

In Figur 11 ist ein Strahlengang eines herkömmlichen Stereo-Mikroskops schematisch dargestellt. Bei dem herkömmlichen Stereo-Mikroskop blickt der Betrachter mit seinen beiden Augen 901 und 902 in ein linkes Okular 903 bzw. ein rechtes Okular 904 und betrachtet dabei beispielsweise ein Objekt, das in einer Objektebene 905 des Mikroskops angeordnet ist. Die Okulare 903 und 904, Bildumkehrprismen 915 und die Tubuslinsen 913 bilden zusammen den Tubus, der funktionell ein binokulares Kepler-Fernrohr darstellt. Der Betrachter nimmt das Objekt mit einem stereoskopischen Raumeindruck wahr, da ein von dem Objekt ausgehendes Strahlenbündel 907, welches bezüglich einer Mittelebene 906 des Mikroskops nach links zu einer Sammellinse 909 des Mikroskops verläuft, links in diese Sammellinse 909 eintritt und in das linke Okular 903 abgebildet wird, während ein von dem Objekt bezüglich der Mittelebene 906 nach rechts ausgehendes Strahlenbündel 908 auch rechts in die Sammellinse 909 eintritt und in das rechte Okular 904 abgebildet wird. Die Objektivanordnung umfasst zur Änderung der Vergrößerung ferner ein afokales Zoomsystem 911, und die Okulare 903, 904 umfassen jeweils eine Tubuslinse 913 und ein Schmidt-Pechan-Prisma 915. Die Schmidt-Pechan-Prismen sind notwendig, um das Bild des Objekts, dessen Bildorientierung durch das Objektiv umgekehrt wurde, wieder höhen- und seitenrichtig zu stellen.

Das herkömmliche Stereo-Mikroskop kann auch in der Augenchirurgie zur Betrachtung eines Augenfundus 916 eines Patientenauges 917 eingesetzt werden, wozu vor dem Patientenauge eine Ophthalmoskopierlinse 919 angeordnet wird, welche in der Objektebene 905 des Mikroskops ein Zwischenbild des Augenfundus 916 erzeugt. Das Zwischenbild des Augenfundus wird dann vom Operateur durch das Stereo-Mikroskop betrachtet.

Bei der in Figur 11 gezeigten Anordnung des Stereo-Mikroskops und der Ophthalmoskopierlinse stellt sich auf Grund der Änderungen der Bildorientierung und stereoskopischen Blickrichtung durch die Ophthalmoskopierlinse der Augenfundus für den Operateur höhen- und seitenverkehrt und zudem pseudo-stereoskopisch dar, d.h. in der Tiefenwahrnehmung ist für den Operateur vorne und hinten vertauscht. Um dieses Problem zu beheben, muss zum einen die richtige Bildorientierung wieder hergestellt werden und zum anderen müssen die beiden Stereokanäle des Mikroskops vertauscht werden, d.h. das von der Objektebene 905 nach links ausgehende Strahlenbündel 907 muss dem rechten Auge 902 des Operateurs zugeführt werden, und das nach rechts ausgehende Strahlenbündel 908 muss dessen linkem Auge 901 zugeführt werden.

Hierzu sind aus dem Stand der Technik eine Reihe von Möglichkeiten bekannt:

Gemäß DE 41 14 646 A1 kann zwischen Ophthalmoskopierlinse und Sammellinse des Objekts ein Schmidt-Pechan-Prisma mit Dachkante angeordnet werden.

Gemäß DE 38 26 069 A1 und der korrespondierenden US 5,009,487 kann in dem parallelen Strahlengang zwischen Vergrößerungswechsler und Tubuslinsen der Okulare ein Spiegelsystem angeordnet werden, welches die Bildorientierung ändert und den rechten mit dem linken Strahlengang vertauscht.

Die US 5,200,773 beschreibt einen Ophthalmoskopievorsatz, der ein für den linken und rechten Strahlengang gemeinsames Porro-Prisma erster Art aufweist, welches eine Bildaufrichtung noch vor der Zwischenbildebene vornimmt. Im Falle einer Nutzung in Verbindung mit einem Stereomikroskop bewirkt die für beide Strahlengänge gemeinsame Bildaufrichtung auch eine Vertauschung des linken und rechten Strahlengangs.

Nachteile bei den bekannten Lösungen sind zum einen eine Behinderung des Zugangs zum Operationsfeld, wenn die Bildvertauschung nahe der Ophthalmoskopierlinse vorgenommen wird, und zum anderen eine ergonomisch ungünstige Vergrößerung der Bauhöhe des Mikroskops, wenn das Spiegelsystem zwischen afokalem Zoomsystem und Tubus angebracht ist.

Es ist eine Aufgabe der vorliegenden Erfindung, eine verbesserte Stereo-Mikroskopieanordnung zur Betrachtung eines Objekts oder eines von einem Objekt erzeugten Zwischenbilds bereitzustellen, welches sich insbesondere zur Betrachtung eines Zwischenbilds mit verkehrter Bildorientierung und Pseudostereoskopie eignet.

Zur Lösung dieser Aufgabe ist erfindungsgemäß eine Stereo-Mikroskopieanordnung vorgesehen, wie sie in dem beiliegenden unabhängigen Anspruch definiert ist. Vorteilhafte Ausgestaltungen der Erfindung sind in den abhängigen Ansprüchen definiert.

Die Erfindung geht aus von einer Stereo-Mikroskopieanordnung mit einer Objektivanordnung und einer Objektebene zur Anordnung eines zu betrachtenden Objekts bzw. Zwischenbilds, wobei ein Strahlvertauschungs- und Bildumkehrsystem zwischen Objektebene und Eintrittslinse der Objektivanordnung angeordnet ist. Das Strahlvertauschungs- und Bildumkehrsystem führt ein bezüglich einer Mittelebene nach links in Richtung zu der Objektivanordnung gerichtetes Strahlenbündel der Objektivanordnung rechts zu, es führt ein bezüglich der Mittelebene nach rechts in Richtung zu der Objektivanordnung gerichtetes Strahlenbündel der Objektivanordnung links zu, und es kehrt die Bildorientierungen der beiden Strahlenbündel jeweils um. Damit ist die Stereo-Mikroskopieanordnung besonders zur Betrachtung eines höhen- und seitenverkehrten Zwischenbilds geeignet.

Vorzugsweise umfaßt das das Strahlvertauschungs- und Bildumkehrsystem wenigstens ein Porro-Prisma zweiter Art.

Es hat sich herausgestellt, dass das Porro-Prisma zweiter Art eine besonders wirksame optische Komponente ist, um einen für die Strahlvertauschung notwendigen Versatz zwischen eintretendem und austretendem Strahlenbündel bereitzustellen und gleichzeitig die gewünschte Bildumkehr zu erzielen. Das Porro-Prisma zweiter Art ist vergleichsweise kompakt mit einer vergleichsweise kurzen optischen Weglänge, so dass eine Behinderung des freien Arbeitsraumes und auch sichtbare Störungen des betrachteten Bildes, wie etwa Bildfeldbeschnitt, weitgehend vermeidbar sind.

Vorzugsweise ist hierbei für einen jeden der beiden Strahlenbündel ein diesem separat zugeordnetes Porro-Prisma zweiter Art vorgesehen, wodurch die Bildumkehr und Strahlvertauschung mit optischen Komponenten erreicht wird, die zum gesamten Lichtweg der Strahlenbündel mit nur einer vergleichsweise kurzen optischen Weglänge beitragen.

Eine hierzu alternative Anordnung ist dann vorteilhaft, wenn die Stereo-Mikroskopieanordnung zur Benutzung durch zwei Beobachter ausgelegt ist. Der Objektivanordnung bezüglich einer Mittelebene links und rechts zugeführte Strahlenbündel werden dabei zu dem linken bzw. rechten Auge eines ersten Beobachters abgebildet, während der Objektivanordnung zentral zur Mittelebene oben und unten zugeführte Strahlenbündel auf das linke bzw. rechte Auge des zweiten Beobachters abgebildet werden.

Hierbei ist vorzugsweise ein erstes Porro-Prisma zweiter Art vorgesehen, um die oben und unten zugeführten Strahlenbündel für den zweiten Beobachter sowie das links zugeführte Strahlenbündel für den ersten Beobachter abzubilden, während ein zweites Porro-Prisma zweiter Art vorgesehen ist, um das rechts zugeführte Strahlenbündel für den ersten Beobachter abzubilden. Hierdurch ist eine einfache Anordnung geschaffen, die für zwei unabhängige Beobachter die Strahlvertauschung und Bildumkehr auf einfache Weise bewerkstelligt.

Die erfindungsgemäße Stereo-Mikroskopieanordnung dient zur abwechselnden Betrachtung der Cornea eines Auges als erstes Objekt, und des Augenfundus des Auges als zweites Objekt. Zur Betrachtung des Zwischenbilds des zweiten Objekts werden in den Strahlengang vor dem Objektiv zum einen ein Umkehr-Linsensystem, beispielsweise Ophthalmoskopievorsatz, zur Erzeugung des höhen- und seitenverkehrten Zwischenbilds des zweiten Objekts und ein Strahlvertauschungs- und Bildumkehrsystem zur Bildumkehr und Pupillenvertauschung des Zwischenbilds eingebracht. Zur Betrachtung des ersten Objekts werden die beiden Komponenten Strahlvertauschungs- und Bildumkehrsystem und Umkehr-Linsensystem aus dem Strahlengang zwischen Auge und Objektiv entfernt.

Erfindungsgemäß ist hierbei vorgesehen, dass der Abstand zwischen dem Objektiv und den Objekten bei der Überführung von dem einen Betrachtungsmodus in den anderen im wesentlichen nicht geändert werden muss, um scharfe Bilder zum einen von dem ersten Objekt, nämlich dem Bereich der Cornea, und zum anderen von dem zweiten Objekt, nämlich dem Bereich des Augenfundus, zu erhalten. Dies wird dadurch erreicht, dass die geometrischen Abmessungen und optischen Medien der Strahlvertauschungs- und Bildumkehranordnung und die Brennweiten des Umkehr-Linsensystems entsprechend aufeinander abgestimmt sind.

Wesentlich ist hierbei, dass vor allem die optische Weglänge des Strahlvertauschungs- und Bildumkehrsystems beispielsweise durch gezielte Auswahl der hierfür verwendeten optischen Medien derart gestaltet ist, dass die Objektebene von dem Objektiv bei in den Strahlengang eingefügten Strahlvertauschungs- und Bildumkehrsystem einen Abstand aufweist, der sich von dem Abstand der Objektebene von dem Objektiv bei aus dem Strahlengang entferntem Strahlvertauschungs- und Bildumkehrsystem um einen Wert unterscheidet, der durch die beabsichtigten Beobachtungspositionen und dabei insbesondere durch Abstände zwischen den zu beobachtenden Objekten und/oder zu deren Beobachtung verwendeter Hilfsmittel bestimmt ist. Im Fall der Augenchirurgie sind die hierfür relevanten Abstände zwischen den zu beobachtenden Objekten im wesentlichen der Abstand zwischen Cornea und dem Zwischenbild des Augenfundus, und die bei der Beobachtung des Objekts verwendeten Hilfsmittel sind im wesentlichen der Ophthalmoskopievorsatz.

Der Abstand zwischen Objektiv und Objektebene bei in den Strahlengang eingefügtem Strahlvertauschungs- und Bildumkehrsystem weist somit eine Differenz zu dem Abstand zwischen Objektiv und Objektebene bei aus dem Strahlengang entferntem Strahlvertauschungs- und Bildumkehrsystem auf. Vorteilhafterweise liegt diese Differenz zwischen den beiden Abständen in einem Bereich von 15 mm bis 40 mm, vorzugweise 20 mm bis 30 mm und weiter bevorzugt 24 mm bis 26 mm.

Vorzugsweise ist vorgesehen, dass das Strahlvertauschungs- und Bildumkehrsystem ein Paar verkürzter Porro-Prismen zweiter Art umfasst. Wie bereits vorangehend schon erläutert, ist das Porro-Prisma zweiter Art besonders dafür geeignet, den für die Strahlvertauschung notwendigen Strahlversatz bei gleichzeitiger Bildumkehr bereitzustellen. Erfindungsgemäß werden weitere Verbesserungen der Bildqualität hierbei dadurch erzielt, dass das herkömmliche Porro-Prisma zweiter Art ersetzt wird durch ein sogenanntes "verkürztes" Porro-Prisma zweiter Art. Dieses verkürzte Porro-Prisma zweiter Art stellt bei gleicher optischer Aufgabe, also Strahlversatz und Bildumkehr, im Vergleich zu dem normalen Porro-Prisma zweiter Art eine verkürzte optische Weglänge bereit, so dass eine Reihe von Beschränkungen durch Bauhöhe und sichtbare Bildstörungen bei der erfindungsgemäßen Ausgestaltung der Stereo-Mikroskopieanordnung vermindert sind.

Im Folgenden werden Ausführungsbeispiele der Erfindung anhand von Zeichnungen näher erläutert. Hierbei zeigt:
- Figur 1: ein Strahlvertauschungssystem zur Verwendung in einer Stereo-Mikroskopieanordnung, wobei die Stereo-Mikroskopieanordnung mit dem Strahlvertauschungssystem allerdings Keine Ausführungsform der Erfindung bildet.
- Figur 2a: eine Teildarstellung des Strahlvertauschungssystems aus Figur 1 in Anordnung in einem konvergenten Strahlengang,
- Figur 2b: den konvergenten Strahlengang aus Figur 2a bei aus dem Strahlengang entferntem Strahlvertauschungssystem und einem dafür eingefügten Bildumkehrsystem,
- Figur 3: eine Teilansicht eines Strahlengangs einer Stereo-Mikroskopieanordnung gemäß einer ersten Ausführungsform der Erfindung,
- Figur 4: eine schematische Darstellung eines Ophthalmoskopievorsatzes zur Verwendung mit der erfindungsgemäßen Stereo-Mikroskopieanordnung,
- Figur 5: eine Darstellung eines Strahlengangs durch ein Porro-Prisma zweiter Art,
- Figur 6a: eine Darstellung des Strahlengangs zwischen Objektebene und Objektiv bei einem aus dem Strahlengang entfernten Strahlvertauschungs- und Bildumkehrsystem bei der Ausführungsform gemäß Figur 3,
- Figur 6b: eine der Figur 6a entsprechende Darstellung mit in den Strahlengang eingesetztem Strahlvertauschungs- und Bildumkehrsystem,
- Figur 6c: eine der Figur 6b entsprechende Darstellung, wobei der Strahlengang durch das Strahlvertauschungs- und Bildumkehrsystem entfaltet dargestellt ist,
- Figur 7: eine Detailansicht eines Strahlengangs durch eine Stereo-Mikroskopieanordnung gemäß einer zweiten Ausführungsform der Erfindung,
- Figur 8: eine schematische Darstellung der Anordnung von Porro-Prismen zweiter Art vor dem Objektiv der in Figur 7 dargestellten Stereo-Mikroskopieanordnung,
- Figur 9: eine räumliche Teildarstellung der in Figur 7 gezeigten Ausführungsform,
- Figur 10: ein verkürztes Porro-Prisma zweiter Art zur Verwendung bei einer weiteren Ausführungsform der erfindungsgemäßen Stereo-Mikroskopieanordnung,
- Figur 11: eine Stereo-Mikroskopieanordnung gemäß dem Stand der Technik und
- Figur 12: eine Seitenansicht einer Stereo-Mikroskopieanordnung gemäß einer weiteren Ausführungsform der Erfindung.

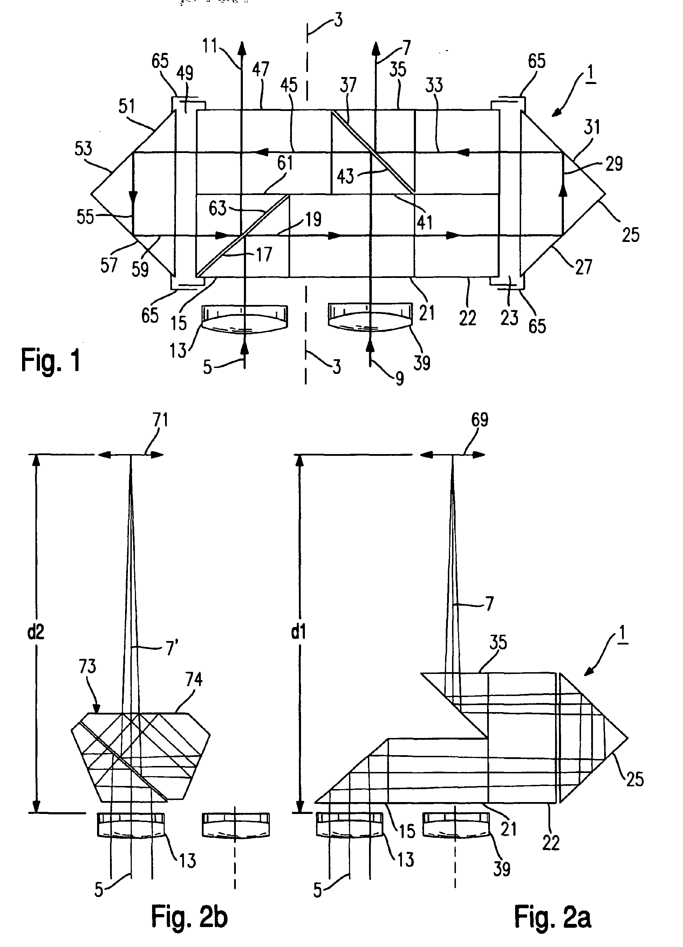

Zunächst ist nachfolgend unter Bezugnahme auf die Figuren 1 und 2 ein Stereo-Mikroskop beschrieben, welches wahlweise in zwei Beobachtungsmodi einsetzbar ist und welches ein Vergleichsbeispiel zu den Ausführungsformen der Erfindung darstellt. In einem ersten Beobachtungsmodus dient das Stereo-Mikroskop der Betrachtung eines höhen- und seitenverkehrten Zwischenbilds, wie es beispielsweise durch einen Ophthalmoskopievorsatz zur Abbildung eines Augenfundus in einer Objektebene des Mikroskops erzeugt wird, und in einem zweiten Beobachtungsmodus dient das Mikroskop der direkten Betrachtung eines in der Objektebene angeordneten Objekts.

Der Ophthalmoskopievorsatz erzeugt für einen Beobachter ein höhen- und seitenverkehrtes Abbild des Augenfundus als Zwischenbild in pseudo-stereoskopischer räumlicher Darstellung. Um diesen störenden Effekt zu vermeiden, ist in den Strahlengang des Stereo-Mikroskops ein Strahlvertauschungssystem 1 gemäß Figur 1 eingefügt. Das Strahlvertauschungssystem überführt ein bezüglich einer Mittelebene 3 links in das Strahlvertauschungssystem eintretendes Strahlenbündel 5 in ein rechts aus diesem austretendes Strahlenbündel 7, und es überführt ein bezüglich der Mittelebene 3 rechts in das Strahlvertauschungssystem 1 eintretendes Strahlenbündel 9 in ein bezüglich der Mittelebene 3 links aus diesem austretendes Strahlenbündel 11. Hierbei weisen die eintretenden Strahlenbündel 5, 9 und die austretenden Strahlenbündel 7, 11 einander gleiche Bildorientierungen auf, d.h. eine Bildumkehr findet durch das Strahlvertauschungssystem 1 nicht statt.

Das Strahlvertauschungssystem 1 ist aus Glasklötzen und Glasprismen aufgebaut, an deren Innenflächen die Strahlenbündel reflektiert werden.

Der Strahlenverlauf in dem Strahlvertauschungssystem 1 ist in Figur 1 anhand des Verlaufs von Zentralstrahlen der Strahlenbündel 5 und 9 dargestellt.

Nach Durchlaufen einer Tubuslinse 13 einer linken Okularanordnung des Stereo-Mikroskops tritt der Strahl 5 in ein 90° Prisma 15 ein. Das 90° Prisma 15 weist ein unter 45° zur Richtung des Strahls 5 derart angeordnete Spiegelfläche 17 auf, dass der Strahl 5 um 90° nach rechts in einen Strahl 19 abgelenkt wird. Der Strahl 19 verlässt das 90°-Prisma 15 und durchsetzt nacheinander zwei Glasblöcke 21 und 22, wobei das 90°-Prisma 15 und die Glasblöcke 21, 22 jeweils verkittet sind. Nach Verlassen des Glasblocks 22 durchsetzt der Strahl 19 einen Luftspalt 23 und tritt daraufhin in ein 180°-Prisma 25 ein. An einer ersten Spiegelfläche 27 des 180°-Prismas 25 wird der Strahl 19 um 90° nach links in einen Strahl 29 reflektiert, der sich somit parallel zu der ursprünglichen Richtung des Strahls 5 ausbreitet und der dann eine zweite Spiegelfläche 31 des 180°-Prismas 25 trifft, welche ihn um weitere 90° nach links in einen Strahl 33 reflektiert, der sich antiparallel zu der Richtung des Strahls 19 ausbreitet. Der Strahl 33 verlässt das 180°-Prisma 25 und durchsetzt zunächst den Luftspalt 23 und sodann den Glasblock 22, woraufhin er in ein 90°-Prisma 35 eintritt, welches mit dem Glasblock 22 verkittet ist. An einer Spiegelfläche 37 des 90°-Prismas 35 wird der Strahl 33 um 90° nach rechts reflektiert und verlässt das 90°-Prisma 35 und damit das Strahlvertauschungssystem 1 als Strahl 7 in der rechten Okularanordnung.

Der Zentralstrahl des rechten Strahlenbündels tritt nach Durchlaufen einer Tubuslinse 39 der rechten Okularanordnung von unten nach oben kommend in den Glasblock 21 ein, durchsetzt diesen und tritt dann in ein 90°-Prisma 41 mit einer Spiegelfläche 43 ein, an welcher der Strahl 9 um 90° nach links in einen Strahl 45 reflektiert wird. Hierbei ist das 90°-Prisma 41 mit dem Glasblock 21 verkittet, und ferner sind die 90°-Prismen 41 und 35 derart aneinandergefügt, dass deren Spiegelflächen 43 und 37 mit lediglich einem geringen Abstand voneinander angeordnet sind. Nach Verlassen des 90°-Prismas 41 durchsetzt der Strahl 45 ein mit dem 90°-Prisma 41 verkitteten Glasblock 47, woraufhin der Strahl 45 einen Luftspalt 49 durchsetzt und in ein 180°-Prisma 51 eintritt. An einer Spiegelfläche 53 des 180°-Prismas 51 wird der Strahl 45 um 90° nach links in einen Strahl 55 reflektiert, der sich somit antiparallel zu der Richtung des Strahls 9 ausbreitet und sodann von einer zweiten Spiegelfläche 57 des 180°-Prismas 53 um weitere 90° nach links in einen Strahl 59 reflektiert wird, sodass er sich antiparallel zu dem Strahl 45 ausbreitet. Nach Verlassen des 180°-Prismas 53 durchsetzt der Strahl 59 den Luftspalt 49 und tritt in ein 90°-Prisma 61 mit einer Spiegelfläche 63 ein, an welcher er um weitere 90° nach links abgelenkt wird und nach nochmaligem Durchsetzen des Glasblocks 47 als Strahl 11 in der linken Okularanordnung das Strahlvertauschungssystem 1 verlässt.

Die optischen Weglängen, die die beiden Strahlenbündel zwischen ihrem Eintritt als Strahl 5 und Austritt als Strahl 7 bzw. Eintritt als Strahl 9 und Austritt als Strahl 11 in dem Strahlvertauschungssystem zurücklegen, sind im wesentlichen gleich, was durch die beschriebene Anordnung der Glasprismen und Glasblöcke erreicht wird. Die Glasblöcke 21 und 47 werden hierbei von beiden Strahlenbündeln durchsetzt, während der Glasblock 22 lediglich von dem Strahlenbündel durchsetzt wird, welches als Strahl 5 in das Strahlvertauschungssystem 1 eintritt und als Strahl 7 aus diesem wieder austritt. Letzterer Glasblock 22 dient hierbei zur Kompensation der optischen Weglänge, den das andere Strahlenbündel zusätzlich durchlaufen muss, da es sich als Strahl 55 über eine gewisse Strecke entgegengesetzt zur ursprünglichen Richtung des Strahls 9 von unten nach oben verläuft, während das Strahlenbündel, das als Strahl 5 eintritt, sich nie in eine Richtung entgegen der ursprünglichen Strahlrichtung ausbreitet.

Für einen Feinabgleich der optischen Weglängen, die die beiden Strahlenbündel in dem Strahlvertauschungssystem 1 zurücklegen, sind die Luftspalte 23 und 49 vorgesehen, welche mittels Einstellvorrichtungen 65 änderbar sind, die das 180°-Prisma 25 an den Glasblock 22 bzw. das 180°-Prisma 57 an den Glasblock 47 und das 90°-Prima 15 koppeln.

Wie aus Figur 2a ersichtlich ist, tritt das linke Strahlenbündel 5 als paralleles Strahlenbündel in die Tubuslinse 13 der linken Okularanordnung ein und wird durch diese in ein konvergentes Strahlenbündel umgeformt, so dass in einer Zwischenbildebene 69 der rechten Stereokanals ein scharfes Zwischenbild in der Okular-Zwischenbildebene (69) entsteht. Die Zwischenbildebene 69 ist im Fokus der Tubuslinse 13 mit einem Abstand d1 angeordnet, der durch den Brechungsindex des für die Prismen 15, 25, 35 und die Glasblöcke 21, 22 verwendeten Glases, deren geometrische Abmessungen sowie die Abmessungen der durchlaufenen Luftstrecken bestimmt ist.

Soll nun das Stereo-Mikroskop in dem anderen Beobachtungsmodus eingesetzt werden, bei dem die Ophthalmoskopierlinse aus dem Strahlengang entfernt ist und das zu betrachtende Objekt in der Objektebene des Mikroskops angeordnet ist, so ist eine Strahlvertauschung durch das Strahlvertauschungssystem 1 nicht mehr notwendig, so dass dieses aus dem Strahlengang zu entfernen ist. Andererseits würde dann das in der Objektebene angeordnete Objekt ohne Umkehrprisma von dem Beobachter höhen- und seitenverkehrt wahrgenommen werden. Für die höhen- und seitenrichtige Darstellung des in der Objektebene angeordneten Objekts, wird deshalb statt des Strahlvertauschungssystems 1 in diesem Beobachtungsmodus in dem Strahlengang ein Bildumkehrsystem 73 angeordnet. Dieses wirkt derart, dass das in der linken Okularanordnung verlaufende Strahlenbündel 5 in der linken Okularanordnung verbleibt und als Strahlenbündel 7' in einer Zwischenbildebene 71 der linken Okularanordnung fokussiert wird, wobei auch das in der rechten Okularanordnung verlaufende Strahlenbündel 9 in dieser verbleibt und dort in einer entsprechenden Zwischenbildebene fokussiert wird. Hierzu umfasst das Bildumkehrsystem 73 jeweils ein Schmidt-Pechan-Prisma mit Dachkante 74 für sowohl die linke als auch die rechte Okularanordnung.

Die geometrischen Abmessungen des Schmidt-Pechan-Prismas 74 sowie der Brechungsindex des für dieses verwendeten Glases bestimmen einen Abstand d2 zwischen der Tubuslinse 13 der linken Okularanordnung und der Zwischenbildebene 71 in dem zweiten Beobachtungsmodus. Durch entsprechende Einstellung der Einstellvorrichtungen 65 der Strahlvertauschungssystem 1 ist es möglich, dass die Abstände d1 und d2 gleich sind, so dass bei einem Wechsel des Beobachtungsmodus eine Nachstellung der Okularanordnung zur Erzielung einer scharfen Abbildung nicht notwendig ist.

Entsprechendes gilt für die Abstände, unter denen eine Zwischenbildebene des rechten Strahlenbündels 9 von der Tubuslinse 39 angeordnet ist, wobei die zugehörigen Strahlengänge in den Figuren 2a und 2b der Übersichtlichkeit halber nicht dargestellt sind.

Im folgenden werden Varianten des in den Fig. 1 und 2 dargestellten Stereo-Mikroskops erläutert. Hinsichtlich ihres Aufbaus und ihrer Funktion einander entsprechende Komponenten sind mit den Bezugszahlen aus den Fig. 1 und 2 bezeichnet, jedoch zur Unterscheidung mit einem zusätzlichen Buchstaben versehen. Zur Erläuterung wird auf die gesamte vorangehende Beschreibung Bezug genommen.

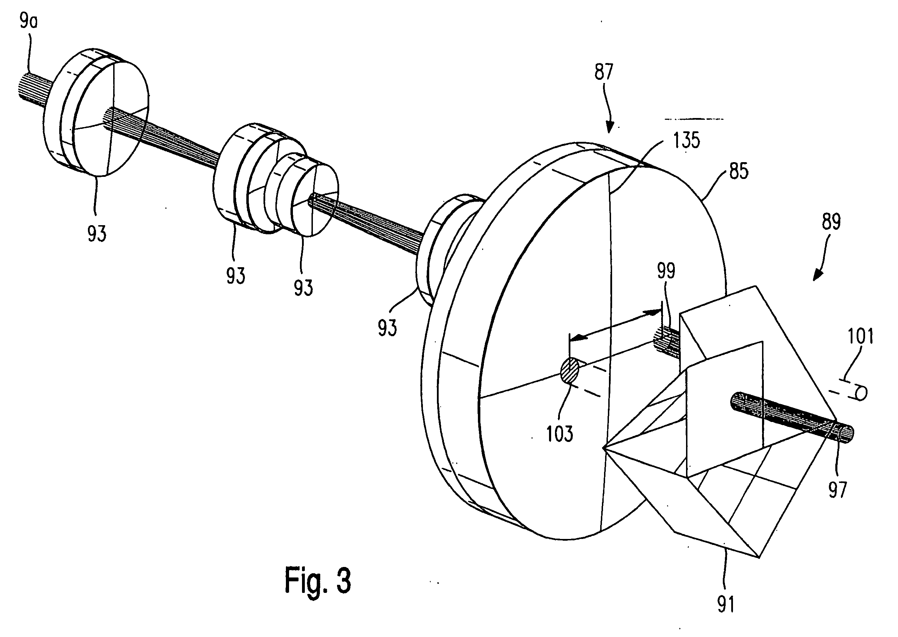

Eine in Figur 3 gezeigte Ausführungsform der Erfindung zeigt eine perspektivische Teilansicht eines Stereo-Mikroskops in dem oben beschriebenen ersten Beobachtungsmodus, d.h. zur Beobachtung eines höhen- und seitenverkehrten Zwischenbilds in der Objektebene des Mikroskops.

Hierzu ist vor einer Eintrittslinse 85 einer Objektivanordnung 87 des Mikroskops ein Strahlvertauschungs- und.Bildumkehrsystem 89 aus zwei Porro-Prismen zweiter Art angeordnet, von denen der Übersichtlichkeit halber nur ein Porro-Prisma 91 in Figur 3 dargestellt ist. Zur Änderung der Vergrößerung umfasst die Objektivanordnung 87 ferner noch ein Zoomsystem mit mehreren Linsen 93. Das Porro-Prisma zweiter Art 91 versetzt ein bezüglich einer Mittelebene 135 der Objektivanordnung 87 links angeordnetes und von der Objektebene her kommendes und auf die Eintrittslinse 85 gerichtetes Strahlenbündel 97 parallel und führt es der Eintrittslinse 85 rechts von der Mittelebene 135 als Strahlenbündel 99 zu. Entsprechend versetzt das zweite in Figur 3 nicht dargestellte Porro-Prisma zweiter Art des Strahlvertauschungs- und Bildumkehrsystems 89 ein von der Objektebene her kommendes und bezüglich der Mittelebene 135 rechts auf die Eintrittslinse 85 gerichtetes Strahlenbündel 101 parallel über die Mittelebene 135 nach links und führt es der Objektivanordnung 87 als Strahlenbündel 103 links zu. Der Verlauf der Strahlenbündel 101 und 103 ist in Figur 3 durch gestrichelte Linien lediglich angedeutet.

Durch die der Objektivanordnung nachfolgende Strahlführung des Mikroskops wird das Strahlenbündel 99 in die rechte Okularanordnung des Mikroskops und entsprechend das Strahlenbündel 103 in die linke Okularanordnung des Mikroskops abgebildet.

Das Strahlvertauschungs- und Bildumkehrsystem 89 umfasst dabei zwei identische Porro-Prismen zweiter Art, welche so ineinandergefügt sind, dass die Spiegelflächen der kleinen 90°-Prismen Rücken an Rücken aneinanderliegen.

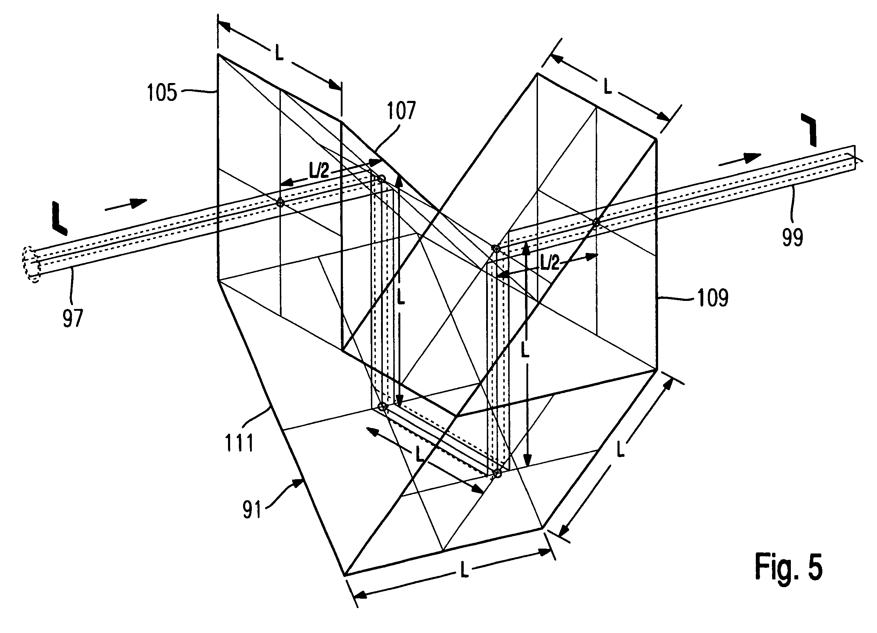

In Figur 5 ist der Strahlverlauf durch das Porro-Prisma zweiter Art 91 vergrößert dargestellt. Dieses Porro-Prisma 91 kann man sich zusammengesetzt denken aus zwei kleinen 90°-Prismen 105 und 109 sowie einem großen 180°-Prisma 111. Die beiden 90°-Prismen 105 und 109 weisen dabei eine kurze Kante der Länge L auf. Der Strahl 97 tritt in das erste 90°-Prisma 105 ein und durchläuft darin eine Strecke von L/2 bis zu seiner Reflexion um 90° an einer Spiegelfläche 107 des Prismas 105. Daraufhin durchläuft der Strahl noch eine Strecke von L/2, bis er in das 180°-Prisma 111 übertritt, worin er wiederum eine Strecke von L/2 bis zu seiner ersten Reflexion um 90° darin zurücklegt. Hiernach durchläuft der Strahl in dem 180°-Prisma 111 eine Strecke der Länge L bis zu seiner zweiten Reflexion darin um weitere 90°, woraufhin der Strahl eine weitere Strecke von L/2 durchläuft, bis er das 180°-Prisma 111 verlässt und in das zweite 90°-Prisma 109 eintritt. Darin durchläuft der Strahl wiederum eine Strecke von L/2 bis zu seiner Reflexion um 90° an der Spiegelfläche des 90°-Prismas 109, welches er nach einer weiteren Strecke von L/2 als Strahl 99 verlässt. Es ist ersichtlich, dass der Strahl in dem Prisma 91 somit eine gesamte geometrische Länge von 4 L zurücklegt.

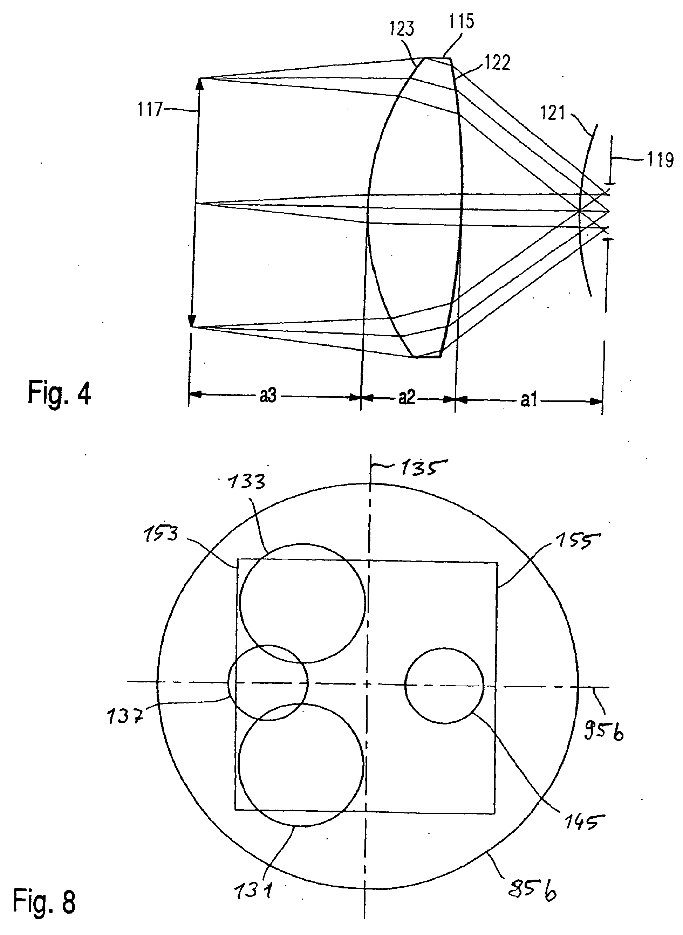

In Figur 4 ist eine zur Verwendung mit dem Stereo-Mikroskop vorgesehene Ophthalmoskopierlinse 115 dargestellt, welche vor einem Auge eines Patienten zur Anordnung kommt, um ein Abbild eines Augenfundus in einer Objektebene 117 des Stereo-Mikroskops zu erzeugen. Von dem zu untersuchenden Auge ist in Figur 4 lediglich schematisch eine Pupillenöffnung 119 und eine Oberfläche einer Cornea 121 dargestellt. Die Linse 115 hat eine dem Auge zu weisende Linsenfläche 122 mit einem Krümmungsradius von 42,474 mm und eine der Objektebene 117 zu weisende asphärische Linsenfläche 123 mit einem Krümmungsradius von 11,372 mm.

Die dem Auge zu weisende Linsenfläche 121 weist in ihrem Zentrum von der Pupille 119 des Auges einen Abstand a1 von etwa 8,5 mm auf. Die beiden Linsenflächen 121 und 123 der Linse 115 sind an ihren Zentren mit einem Abstand a2 von 5,6 mm voneinander angeordnet, während die Linsenfläche 123 von der Objektebene 117 mit einem Abstand a3 von 9,99 mm angeordnet ist. Somit weist die Pupille 119 von der Zwischenbildebene einen Abstand von etwa 24,09 mm auf, wobei die Fläche der Cornea 21 von der Pupillenebene typischerweise etwa 1 mm entfernt ist.

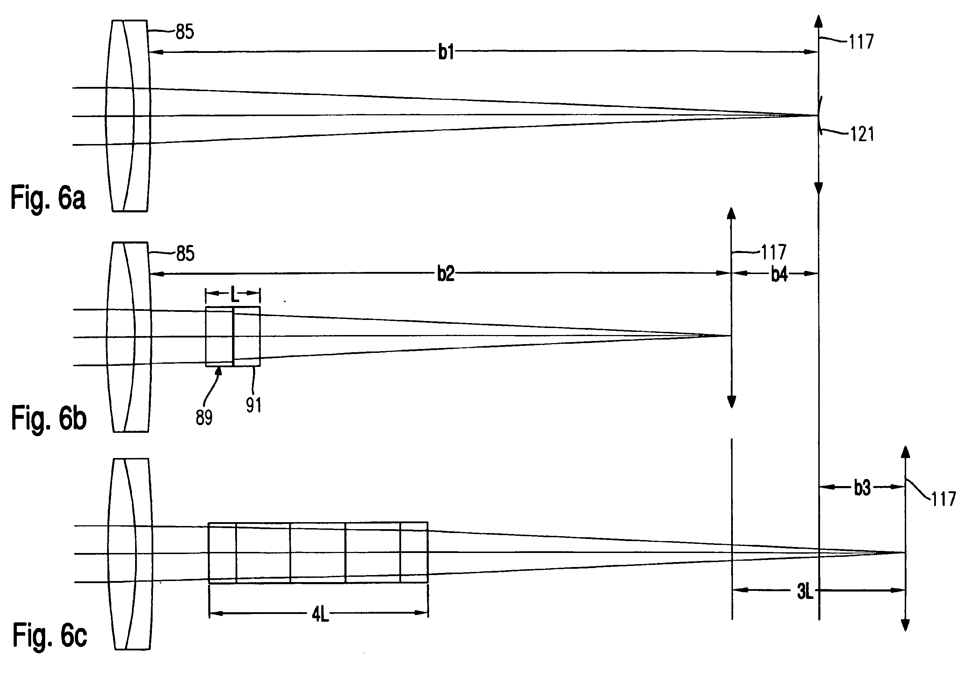

In Figur 6a ist der Strahlenverlauf und die Anordnung der Objektebene 117 des Stereo-Mikroskops in dem zweiten Beobachtungsmodus dargestellt, d.h. in dem Modus, in dem das Strahlvertauschungs- und Bildumkehrsystem 89 aus dem Strahlengang entfernt ist und in dem mit dem Mikroskop die Cornea des zu operierenden Auges betrachtet wird. Es ist somit auch die Cornea 121 in der Objektebene 117 angeordnet. Der Abstand zwischen der Eintrittslinse 85 und der Objektebene 117 beträgt b1.

In Figur 6b ist die Anordnung in dem ersten Beobachtungsmodus dargestellt, in dem das Strahlvertauschungs- und Bildumkehrsystem 89 vor der Eintrittslinse 85 angeordnet ist, um das von der Ophthalmoskopierlinse 115 erzeugte höhen- und seitenverkehrte, pseudostereoskopische Zwischenbild des Augenfundus in der Objektebene 117 des Mikroskops zu beobachten.

Dieses Zwischenbild befindet sich nach Fig. 4 im Abstand B4 = a1 + a2 + a3 vor der Augenpupille. Auf diese Zwischenbildebene muss also im ersten Beobachtungsmodus mit dem Mikroskop fokussiert werden, sie stellt also im ersten Beobachtungsmodus die neue Objektebene für das Mikroskop dar.

Aufgrund der erfindungsgemäßen Ausführung des Porro-Prismas zweiter Art 91 kann nun die mit dem Mikroskop beobachtete Objektebene 117 im ersten Beobachtungsmodus exakt in einen Abstand b2 von der Eintrittslinse 85 verschoben werden, so dass die Differenz b1 - b2 = b4 ist. Somit muss beim Wechsel zwischen den beiden Beobachtungsmodi für eine optimale Bildschärfe nicht nachfokussiert werden.

Zur Erläuterung dieser Bedingung für die Differenz b1 - b2 ist in Fig. 6c der Strahlengang in dem ersten Beobachtungsmodus derart dargestellt, dass der Strahlenverlauf durch das Porro-Prisma 2. Art 91 entfaltet gezeigt ist.

Einerseits wird nun durch einen Glasblock der Länge 4L aus optischen Gründen die Objektebene 117 um b3 = 4L * (n-1)/n verschoben.

Andererseits wird die Objektebene durch die Faltung des Strahlengangs aus mechanischen Gründen um 3L in die entgegengesetzte Richtung verschoben.

Für die Verschiebung der Objektebene um b4 = 3L - b3 in die von der Ophthalmoskopierlinse gegebene Zwischenbildlage des Augenfundus sind also sowohl die Brechzahl n vom Prismenglas als auch die Kantenlänge L des Prismas maßgebend.

Beispielhaft soll hier eine Verschiebung um b4 = 25.0 mm angegeben werden.

Bei einer Bemessung der Kantenlänge L des Prismas 91 zu 16.84 mm und bei Verwendung eines Glases mit der Typenbezeichnung N-SK2 der Firma Schott mit einem Brechungsindex n = 1.60994 ergibt sich für den Abstand B3 ein Wert von 25.52 mm, so dass sich mit 3L = 50.52 mm eine Verschiebung der Objektebene b4 = 3L - b3 von 25.0 mm ergibt.

Durch Änderung des Glases und der Kantenlänge des Prismas kann dieser Wert in einer für die praktischen Erfordernisse ausreichenden Weise variiert werden.

Dies bedeutet, dass beim Wechsel von dem zweiten Beobachtungsmodus, bei dem das Mikroskop auf die Cornea des Auges scharf eingestellt ist, in den ersten Beobachtungsmodus, bei dem die Ophthalmoskopierlinse 115 vor dem Auge angeordnet ist, um das Abbild des Augenfundus zu beobachten, im Wesentlichen keine Änderung des Abstands zwischen der Objektivlinse 85 und dem Auge notwendig ist, was für den Operateur eine große Erleichterung darstellt.

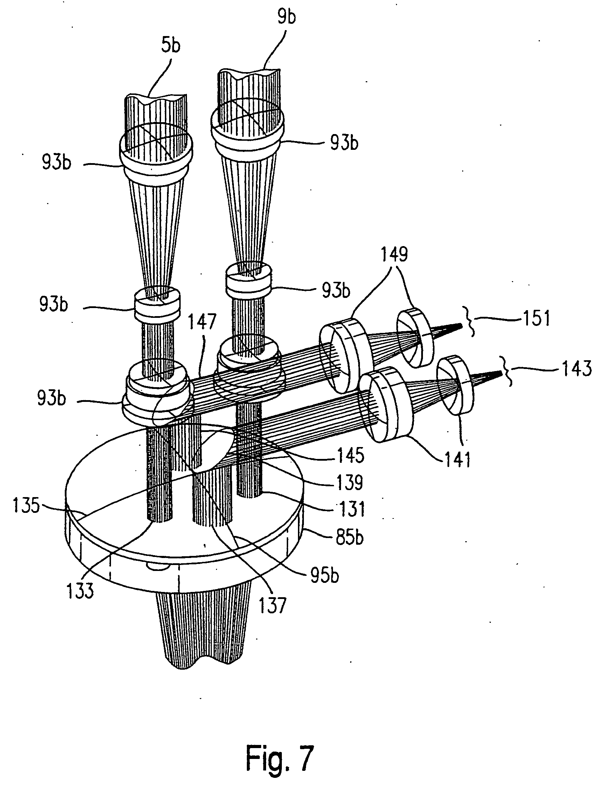

In den Figuren 7 bis 9 ist eine Variante der vorangehend erläuterten Ausführungsform dargestellt, die sich von dieser hauptsächlich dadurch unterscheidet, dass das Stereo-Mikroskop nicht nur für einen Beobachter vorgesehen ist, d.h. mit einem Paar Okularanordnungen versehen ist, sondern dass das Mikroskop vielmehr für zwei Beobachter vorgesehen ist, weshalb zwei Paare von stereoskopischen Beobachtungskanälen mit jeweils zwei Okularen vorgesehen sind.

Figur 7 ist eine räumliche Teildarstellung des Strahlenverlaufs dieses Mikroskops, welche zeigt, wie die entsprechenden Strahlenbündel an die beiden Paare von Okularanordnungen zugeführt werden.

In Figur 7 ist ein Strahlenbündel 5b gezeigt, welches einem linken Okular eines zweiten Beobachters zugeführt wird, sowie ein Strahlenbündel 9b, welches einem rechten Okular des zweiten Beobachters zugeführt wird. Das Strahlenbündel 5b tritt als Strahlenbündel 133 oberhalb von einer Horizontalebene 95b einer Objektivlinse 85b des Mikroskops aus, wobei es in ein Zoomsystem aus Linsen 93b eintritt und dieses als Strahlenbündel 5b verlässt. Entsprechend tritt unterhalb der Horizontalebene 95b ein Strahlenbündel 131 aus der Objektivlinse 85b aus, welches ebenfalls Zoomlinsen 93b des rechten Stereokanals des zweiten Mitbenutzers durchläuft und als Strahlenbündel 9b dann in das rechte Okular des zweiten Beobachters eintritt.

Senkrecht zu der Horizontalebene 95b ist auf der Eintrittslinse 85 in Figur 7 eine Mittelebene 135 dargestellt, so dass die Horizontalebene 95b und die Mittelebene 135 die Eintrittslinse 85b in gleichförmige Quadranten aufteilen.

Ein links von der Mittelebene 135 zentral auf der Horizontalebene 95b austretendes Strahlenbündel 137 trifft auf einen unter 45° zur Ausbreitungsrichtung des Strahlenbündels 137 angeordneten Spiegel 139, welcher das Strahlenbündel 137 um 90° zu seiner ursprünglichen Strahlrichtung reflektiert. Der Strahl 137 durchläuft daraufhin ein Galileisystem aus Linsen 141 und wird als Strahlenbündel 143 einem linken Okular eines ersten Beobachters zugeführt. Ein rechts von der Mittelebene 135 und zentral auf der Horizontalebene 95b aus der Eintrittslinse 85b austretendes Strahlenbündel 145 wird an einem parallel zu dem Spiegel 139 angeordneten weiteren Spiegel 147 ebenfalls um 90° reflektiert und durchsetzt eine weitere Galileianordnung aus Linsen 149, welche parallel zu der Galileianordnung aus den Linsen 141 für den Strahl 137 angeordnet ist, worauf das Strahlenbündel 145 dann als Strahl 151 einem rechten Okular des ersten Beobachters zugeführt wird.

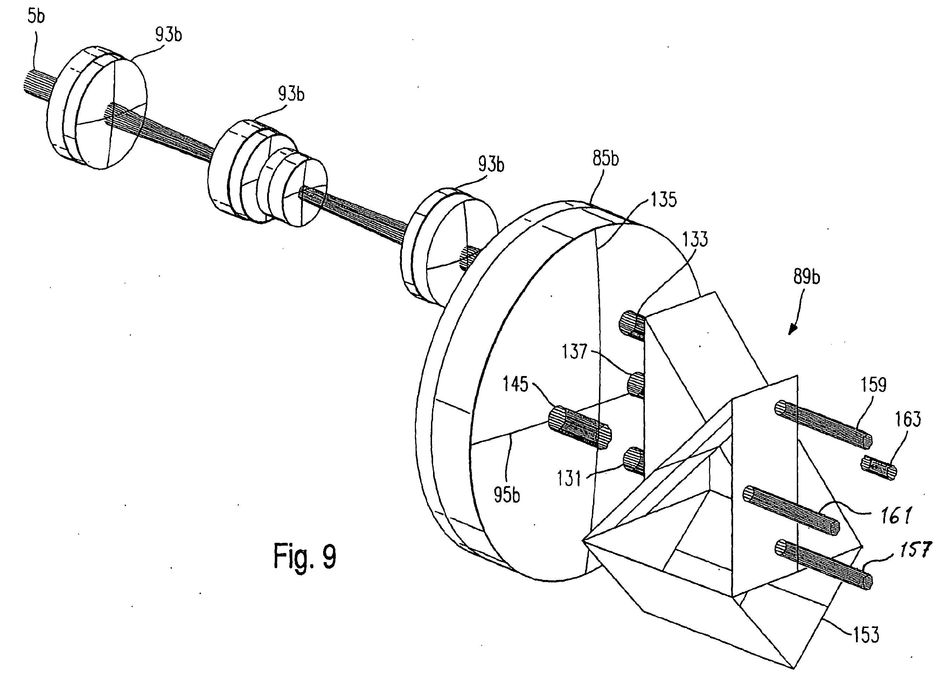

In Figur 8 sind Projektionen der Querschnitte der Strahlenbündel 131, 133, 137 und 145 in Draufsicht auf die von der Objektebene wegweisende Linsenfläche der Linse 85b nochmals im Querschnitt dargestellt, wobei die Bündel 131 und 133 für die bei hoher Vergrößerung auftretenden großen Bündelquerschnitte dargestellt sind.

Aus der perspektivischen Teildarstellung der Figur 9 sind die Strahlenbündel 131, 133, 137 und 145 zu entnehmen, wie sie in die Eintrittslinse 85b des Objekts eintreten. Von den austretenden Strahlenbündeln ist lediglich das Strahlenbündel mit zugehörigen Zoomlinsen 93b gezeigt, welches sich als Strahlenbündel 5b in das linke Okular des zweiten Beobachters fortsetzt. Vor ihrem Eintritt in die Eintrittslinse 85b haben die Strahlenbündel 131, 133, 137 und 145 ein Strahlvertauschungs- und Bildumkehrsystem 89b durchlaufen, welches für die Betrachtung eines höhen- und seitenverkehrten Zwischenbilds in der Objektebene sowohl die notwendige Strahlvertauschung als auch die Bildumkehr bereitstellt.

Das Strahlvertauschungs- und Bildumkehrsystem 89b umfasst zwei Porro-Prismen zweiter Art 153 und 155. In der perspektivischen Darstellung der Figur 9 ist lediglich das Prisma 153 dargestellt, während aus der Figur 8 Projektionen beider Prismen 153 und 155 sichtbar sind.

Das Prisma 153 bewerkstelligt die Strahlvertauschung und Bildumkehr für die beiden den Okularen des zweiten Beobachters zugeführten Strahlenbündel 5b und 9b. Hierzu wird durch das Prisma 153 ein von der Objektebene her kommendes und unterhalb der Horizontalebene 95b auf die Eintrittslinse 85b gerichtetes Strahlenbündel 157 durch das Prisma 153 derart versetzt, dass es das Prisma 153 als das Strahlenbündel 133 verlässt, welches oberhalb der Horizontalebene 95b in das Objektiv eintritt und als Strahlenbündel 5b für das linke Auge des zweiten Beobachters bestimmt ist. Entsprechend tritt ein oberhalb der Horizontalebene 95b auf die Eintrittslinse 85b gerichtetes Strahlenbündel 159 in das Prisma 153 ein und aus diesem als Strahlenbündel 131 wieder aus, welches für das rechte Auge des zweiten Beobachters bestimmt ist.

Weiterhin tritt in das Prisma 153 ein Strahlenbündel 161 ein, welches zentral auf die Horizontalebene 95b und rechts der Mittelebene 135 auf die Eintrittslinse 85b gerichtet ist. Dieses Strahlenbündel 161 wird durch das Prisma 153 derart versetzt, dass es als das Strahlenbündel 137 zentral auf der Horizontalebene 95b und links von der Mittelebene 135 in das Objektiv einfällt, wie dies auch aus Figur 8 ersichtlich ist. Wie vorangehend bereits erläutert, ist das Strahlenbündel 137 für das linke Auge des ersten Beobachters bestimmt.

In das zweite Prisma 155 des Strahlvertauschungs- und Bildumkehrsystems 89b tritt lediglich ein Strahlenbündel 163 ein, dessen räumliche Anordnung bezüglich der anderen Strahlenbündel 157, 159 und 161 aus Figur 9 ersichtlich ist. Das Strahlenbündel 163 ist bezüglich der Horizontalebene mittig links von der Mittelebene 135 auf die Eintrittslinse 85b des Objektivs gerichtet. Durch das Prisma 155 wird das Strahlenbündel 163 derart versetzt, dass es als das Strahlenbündel 145 bezüglich der Horizontalebene 95b mittig und rechts von der Mittelebene 135 in das Objektiv eintritt, wie dies in Figur 9 ebenfalls andeutungsweise dargestellt ist.

In der Darstellung der Figur 8 sind die projizierten Austrittsflächen der beiden Prismen 153 und 155 dargestellt. Aus der Figur 8 ist damit ersichtlich, dass aus dem Porro-Prisma zweiter Art 153 die beiden für das linke und das rechte Auge des zweiten Beobachters bestimmten Strahlenbündel 131 und 133 sowie das für das linke Auge des ersten Beobachters bestimmte Strahlenbündel 137 austreten. Aus dem Prisma 155 tritt lediglich das für das rechte Auge des ersten Beobachters bestimmte Strahlenbündel 145 aus.

Mit dieser Anordnung ist ein vor dem Objektiv des Mikroskops anordenbares Strahlvertauschungs- und Bildumkehrsystem realisiert, welches vergleichsweise wenig Bauraum einnimmt und damit den Zugang zum Operationsfeld verhältnismäßig wenig stört. Zudem erfolgt die Strahlvertauschung und Bildumkehr für den zweiten Beobachter durch ein integrales Prisma, so dass ein Problem der Justierung der Bildlagen für das linke und das rechte Auge relativ zueinander für diesen Beobachter nicht auftritt. Allerdings sind auch zwei identische Porro-Prismen zweiter Art mit solcher Präzision ineinandersteckbar und relativ zueinander justierbar, dass auch für den ersten Benutzer, dessen beide Stereokanäle beide Prismen benutzen, Bildlagenprobleme weitgehend vermeidbar sind.

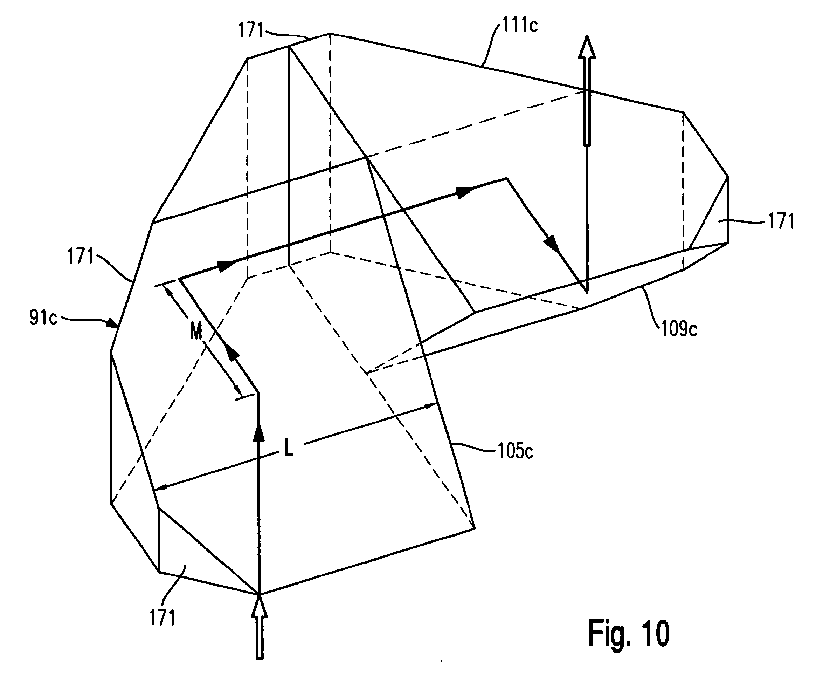

In Figur 10 ist ein an sich bekanntes "verkürztes" Porro-Prisma zweiter Art 91c dargestellt. Auch dieses Prisma kann man sich zusammengesetzt denken aus zwei 90°-Prismen 105c und 109c und einem 180°-Prisma 111c. Im Unterschied zu dem in Figur 5 gezeigten Porro-Prisma zweiter Art ist bei dem Prisma 91c der Figur 10 das 180°-Prisma 111c in seiner Scheitelhöhe verkürzt, so dass dessen reflektierende Flächen näher an die 90°-Prismen 105c und 109c herangerückt sind. Zudem sind bei sämtlichen Teilprismen 105c, 109c, 111c des Prismas 91c Kanten 171 gebrochen, welche von dem zu spiegelnden Strahlenbündel nicht durchsetzt werden. Wie aus Figur 10 ersichtlich ist, legt ein Zentralstrahl zwischen seiner Reflexion an dem 90°-Prisma 105c und seiner ersten Reflexion an dem 180°-Prisma 111c eine Strecke M zurück, welche sich berechnet zu M = 0,71 L, wobei L wieder eine Kantenlänge des 90°-Prismas 105c, 109c darstellt.

Somit weist die in dem Prisma 91c zurückgelegte Strecke eines Strahls einen Wert von 3,42 L auf, was im Vergleich zu dem Wert von 4 L für das Prisma der Figur 5 eine verkürzte optische Weglänge bedeutet. Gleichzeitig wirkt das Prisma 91c ebenfalls als Strahlvertauschungs- und Bildumkehrsystem, welches in einem Stereo-Mikroskop zur Betrachtung eines höhen- und seitenverkehrten Zwischenbildes einsetzbar ist.

Das verkürzte Porro-Prisma zweiter Art kann an verschiedenen Stellen des Mikroskops eingesetzt werden. Hierbei kommt eine Anordnung vor dem Objektiv in Betracht, wie das für die normalen Porro-Prismen zweiter Art bereits in Zusammenhang mit den in den Figuren 3 und 9 dargestellten Ausführungsformen erläutert wurde.

Weiterhin ist das verkürzte Porro-Prisma zweiter Art zur Strahlvertauschung und Bildumkehr im Strahlengang zwischen dem Objektiv und den Okularen einsetzbar, also bei einem Vergleich mit der eingangs bereits erläuterten Figur 11 zwischen den Elementen 909 und 913. Hierbei ist schließlich eine Anordnung zwischen einem Vergrößerungs-Änderungssystem und den Okularen möglich (zwischen den Komponenten 911 und 913 in Figur 11), besonders bevorzugt ist aber eine Anordnung zwischen dem Objektiv bzw. der Sammellinse des Objektivs und dem Vergrößerungs-Änderungssystem (zwischen den Komponenten 909 und 911 in Figur 11). Diese Anordnung ist deshalb besonders bevorzugt, weil dort eine Störung des erzeugten Bildes, wie etwa Vignettierung, in vergleichsweise geringem Umfang auftritt.

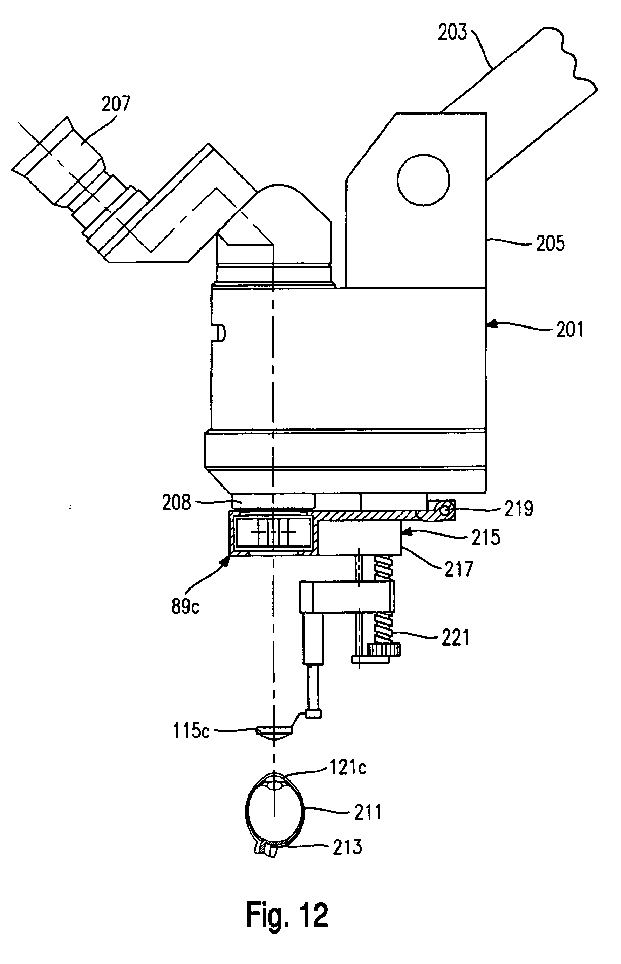

In Figur 12 ist eine Seitenansicht eines Stereo-Mikroskops 201 dargestellt, welches mit einem Strahlvertauschungs- und Bildumkehrsystem 89c versehen ist. Ein Mikroskopkörper 205 ist an einem Stativ 203 verschenkbar gehaltert und trägt ein Paar Okulare 207 und ein Objektiv 208, wobei die in den Okularen 207, dem Objektiv 208 sowie weiter in dem Mikroskopkörper 205 enthaltenen Linsensysteme in Figur 12 nicht dargestellt sind. Diese Linsensysteme können einen Aufbau aufweisen, wie er in den Figuren 3 bis 11 erläutert wurde, wobei auch andere Linsenanordnungen möglich sind. Das Stereo-Mikroskop 201 ist zur Beobachtung eines Patientenauges 211 vorgesehen, und zwar soll ein Augenfundus 213 durch eine Cornea 121c des Auges beobachtet werden. Hierzu weist das Mikroskop 201 ein an dem Mikroskopkörper 205 gehaltertes Vorsatzsystem 215 auf. Als optische Komponenten umfaßt das Vorsatzsystem 215 das Strahlvertauschungs- und Bildumkehrsystem 89c, welches vor dem Objektiv 208 zur Anordnung kommt, und eine Ophtalmoskopierlinse 115c, welche zwischen dem Strahlvertauschungs- und Bildumkehrsystem 89c und der Cornea 121c zur Anordnung kommt. Das Strahlvertauschungs- und Bildumkehrsystem 89c und die Ophtalmoskopierlinse 115c sind an einer gemeinsamen Halterung 217 befestigt, welche wiederum an dem Mikroskopkörper 205 gehaltert ist. Hierbei ist die Halterung 217 um eine Achse 219 schwenkbar an dem Mikroskopkörper 205 gehaltert, so daß das optische System aus Strahlvertauschungs- und Bildumkehrsystem 89c und Ophtalmoskopierlinse 115 auf einfache Weise in den Strahlengang des Mikroskops einschwenkbar und aus diesem wieder entfernbar ist. Im Hinblick auf eine einfache Abstimmung des von dem Mikroskop 201 erzeugten Bildes des Augenfundus 213 ist der Abstand zwischen Ophtalmoskopierlinse 115 und dem Strahlvertauschungs- und Bildumkehrsystem 89c über einen Spindeltrieb 221 änderbar.

In dem vorangehend beschriebenen Ausführungsbeispiel der Figur 12 sind das Strahlvertauschungs- und Bildumkehrsystem und die Ophtalmoskopierlinse 115 gemeinsam in den Strahlengang des Mikroskops einschwenkbar gehaltert. Es ist jedoch ebenfalls möglich, die Ophtalmoskopierlinse separat von dem Mikroskop, beispielsweise an einem Stativ, zu haltern und das Strahlvertauschungs- und Bildumkehrsystem schwenkbar an dem Mikroskop zu haltern. Ebenso ist es möglich, von der in Figur 12 gezeigten schwenkbaren Halterung abzuweichen und beispielsweise eine oder mehrere anders orientierte Schwenkachsen vorzusehen. Ferner ist es möglich, das Vorsatzsystem beispielsweise an einem Schlitten zu haltern, um es in den Strahlengang hineinzuschieben und aus diesem auch wieder herauszuschieben.

Das in Figur 1 gezeigte Strahlvertauschungssystem ist bei der dort beschriebenen Ausführungsform im Strahlengang der Okularanordnungen des Stereo-Mikroskops angeordnet. Allerdings ist dieses Strahlvertauschungssystem auch an jeder anderen Stelle des Strahlengangs des Stereo-Mikroskops einsetzbar, um die gewünschte Wirkung, nämlich eine Strahlvertauschung unter Beibehaltung der Bildorientierung zu erzielen.

Ferner ist der Einsatz dieses Strahlvertauschungssystems, also der in Zusammenhang mit Figur 1 beschriebenen Anordnung, von zwei 90°-Spiegeln und einem 180°-Doppelspiegel pro Strahlengang nicht auf die Anwendung in Stereo-Mikroskopen beschränkt. Es ist die Verwendung dieses Strahlvertauschungssystems vielmehr für jeden anderen Bereich der Optik vorgesehen, in dem das Problem einer Strahlvertauschung unter Beibehalten der Bildorientierung auftritt.

In den vorangehenden Ausführungsformen wurden jeweils die Verwendung einer Ophthalmoskopierlinse zur Erzeugung eines höhen- und seitenverkehrten Zwischenbilds eines Augenfundus beschrieben, um das Zwischenbild in der Objektivebene des Mikroskops zu erzeugen. Allerdings ist das beschriebene Mikroskop für jede andere Anwendung vorgesehen, bei der das Problem der Betrachtung eines höhen- und seitenverkehrten Zwischenbilds in der Objektebene des Mikroskops auftritt. Dies können beispielsweise andere chirurgische Anwendungen oder jeglicher anderer Einsatz sein, für den die Verwendung eines Stereo-Mikroskops in Betracht kommt.

## Patentansprüche

1. Stereo-Mikroskopieanordnung zur Betrachtung eines Augenfundus eines Auges mit einer Ophthalmoskopierlinse in einem ersten Beobachtungsmodus und einer Cornea des Auges in einem zweiten Beobachtungsmodus, umfassend:
eine Objektivanordnung (87) mit einer Objektebene (117) zur Anordnung des zu betrachtenden Objekts bzw. Zwischenbilds, und ein Strahlvertauschungs- und Bildumkehrsystem (89),
**dadurch gekennzeichnet, dass**
das Strahlvertauschungs- und Bildumkehrsystem (89) ein von der Objektebene (117) nach links in Richtung zu der Objektivanordnung (87) gerichtetes Strahlenbündel (101, 163) der Objektivanordnung (87) rechts zuführt, ein von der Objektebene (117) nach rechts in Richtung zu der Objektivanordnung (87) gerichtetes Strahlenbündel (97, 161) der Objektivanordnung (87) links zuführt, und Bildorientierungen der beiden Strahlenbündel (97, 101, 161, 163) jeweils umkehrt,
wobei das Strahlvertauschungs- und Bildumkehrsystem (89) für den zweiten Beobachtungsmodus aus dem Strahlengang vor der Objektivanordnung (87) entfernbar ist, wobei bei in dem Strahlengang angeordnetem Strahlvertauschungs- und Bildumkehrsystem (89) die Objektebene (117) der Objektivanordnung (87) von der Objektivanordnung (87) einen Abstand b2 aufweist, wobei bei aus dem Strahlengang entferntem Strahlvertauschungs- und Bildumkehrsystem (89) die Objektebene (117) von der Objektivanordnung (89) einen Abstand b1 aufweist, und wobei eine Differenz zwischen dem Abstand b1 und dem Abstand b2 einen derart vorbestimmten Wert aufweist, daß beim Wechsel zwischen den Beobachtungsmodi im Wesentlichen keine Anderung des Abstands zwischen der Objektivanordnung (87) und dem Auge notwendig ist, um scharfe Bilder zum einen von der Cornea und zum anderen von dem Augenfundus zu erhalten.

2. Stereo-Mikroskopieanordnung nach Anspruch 1, wobei das Strahlvertauschungs- und Bildumkehrsystem (89) wenigstens ein Porro-Prisma zweiter Art (91; 153) umfasst.

3. Stereo-Mikroskopieanordnung nach Anspruch 1, wobei das Strahlvertauschungs- und Bildumkehrsystem (89) zwei zu einer symmetrischen Anordnung ineinandergefügte verkürzte Porro-Prismen zweiter Art (91c) umfaßt, wobei die beiden verkürzten Porro-Prismen zweiter Art (91c) jeweils eine gegenüber einem nicht-verkürzten Porro-Prisma zweiter Art (91) kleinere Scheitelhöhe aufweisen.

4. Stereo-Mikroskopieanordnung nach Anspruch 2, wobei für einen ersten Beobachter zwei erste Okularanordnungen vorgesehen sind, denen die der Objektivanordnung (87) links (99, 137) und rechts (97, 161) zugeführten Strahlenbündel zugeführt werden.

5. Stereo-Mikroskopieanordnung nach Anspruch 4, ferner umfassend ein im Strahlengang zwischen den ersten Okularanordnungen und der Objektivanordnung (87) angeordnetes erstes Vergrößerungsänderungssystem (93).

6. Stereo-Mikroskopieanordnung nach Anspruch 4 oder 5, wobei für einen zweiten Beobachter zwei weitere Okularanordnungen vorgesehen sind, denen ein der Objektivanordnung (87) oben zugeführtes Strahlenbündel (133) und ein der Objektivanordnung (87) unten zugeführtes Strahlenbündel (131) zugeführt werden.

7. Stereo-Mikroskopieanordnung nach Anspruch 6, ferner umfassend ein zwischen den weiteren Okularanordnungen und der Objektivanordnung (87) angeordnetes zweites Vergrößerungsänderungssystem (93b).

8. Stereo-Mikroskopieanordnung nach Anspruch 6 oder 7, wobei eines der beiden Porro-Prismen zweiter. Art (153,155) sowohl ein von der Objektebene (117) bezüglich einer zu einer Mittelebene (135) der Objektivanordnung (87) senkrechten Horizontalebene (95b) nach unten in Richtung zu der Objektivanordnung (87) gerichtetes Strahlenbündel (157) der Objektivanordnung (87) unter Umkehrung von Bildorientierungen als das dieser oben zugeführte Strahlenbündel (133) zuführt, als auch ein von der Objektebene (117) bezüglich der Horizontalebene (95b) nach oben in Richtung zu der Objektivanordnung (87) gerichtetes Strahlenbündel (159) der Objektivanordnung (87) unter Umkehrung von Bildorientierungen als das dieser unten zugeführte Strahlenbündel (131) zuführt.

9. Stereo-Mikroskopieanordnung einem der Ansprüche 1 bis 8, wobei das Strahlvertauschungs- und Bildumkehrsystem (89) in den Strahlengang einschwenkbar oder einschiebbar ist.

10. Stereo-Mikroskopieanordnung nach einem der Ansprüche 1 bis 9 ferner umfassend ein Ophthalmoskopierlinsensystem (115) zur Erzeugung eines Zwischenbilds eines Augenfundus (213) in der Objektebene (117) der Objektivanordnung (87), wobei das Ophthalmoskopierlinsensytem (115) gemeinsam mit dem Strahlvertauschungs- und Bildumkehrsystem (89) aus dem Strahlengang entfernbar und wieder einfügbar ist.

11. Stereo-Mikroskopieanordnung nach einem der Ansprüche 1 bis 9, ferner umfassend ein Ophtalmoskopierlinsensystem (115) zur Erzeugung eines Zwischenbilds eines Augenfundus (213) in der Objektebene (117) der Objektivanordnung (87), wobei das Strahlvertauschungs- und Bildumkehrsystem (89) und das Ophtalmoskopierlinsensysteme (115) aus dem Strahlengang vor der Objektivanordnung(87) entfernbar sind, und wobei geometrische Abmessungen und optische Medien der Strahlvertauschungs- und Bildumkehr-Anordnung (89) und Brennweiten des Ophthalmoskopierlinsensystems (115) derart abgestimmt sind, daß bei in dem Strahlengang angeordnetem Strahlvertauschungs- und Bildumkehrsystem (89) und Ophthalmoskopierlinsensystem (115) das Zwischenbild des Augenfundus (213) in der Objektebene (117) der Objektivanordnung (87) anordenbar ist und dabei die Objektivanordnung (87) von dem Auge (211) einen Abstand b2+b4 aufweist, und daß bei aus dem Strahlengang entfernten Strahlvertauschungs- und Bildumkehrsystem (89) und Opthtalmoskopierlinsensystem (115) die Cornea (121) des Auges (211) in der Objektebene (117) der Objektivanordnung (87) anordenbar ist und dabei die Objektivanordnung (87) von dem Auge (211) einen Abstand b1 aufweist, und daß der Abstand b2+b4 und der Abstand b1 im wesentlichen gleich sind.

12. Stereo-Mikroskopieanordnung nach einem der Ansprüche 1 bis 11, wobei die Differenz zwischen dem Abstand b1 und dem Abstand b2 15 mm bis 40 mm, vorzugsweise 20 mm bis 30 mm, und weiter bevorzugt 24 mm bis 26 mm beträgt.

## Claims

1. Stereomicroscopy arrangement for viewing an eye fundus by means of an ophthalmoscopy lens in a first viewing mode and for viewing a cornea of the eye in a second viewing mode, comprising:
an objective arrangement (87) with an object plane (117) for positioning the object or intermediate image to be viewed, and a beam interchanging and image inverting system (89),
**characterized in that**
the beam interchanging and image inverting system (89) supplies a beam bundle (101, 163) directed to the left from the object plane (117) in the direction of the objective arrangement (87) to the right of the objective arrangement (87) and supplies a beam bundle (97, 161) directed to the right from the object plane (117) in the direction of the objective arrangement (87) to the left of the objective arrangement (87) and inverts image orientations of the two beam bundles (97, 101, 161, 163),
wherein the beam interchanging and image inverting system (89) is removable for the second viewing mode from the beam path in front of the objective arrangement (87), wherein, when the beam interchanging and image inverting system (89) is positioned in the beam path, the object plane (117) of the objective arrangement (87) is spaced apart from the objective arrangement (87) by a distance b2, and wherein, when the beam interchanging and image inverting system (89) is removed from the beam path, the object plane (117) is spaced apart from the objective arrangement (87) by a distance b1, a difference between the distances b1 and b2 having a value which is predetermined such that, when a change-over between the viewing modes is performed, substantially no change of the distance between the object arrangement (87) and the eye is necessary to obain sharp images of the cornea, on the one hand, and the eye fundus, on the other hand.

2. Stereomicroscopy arrangement according to claim 1, wherein the beam interchanging and image inverting system (89) comprises at least one Porro prism of the second kind (91; 153).

3. Stereomicroscopy arrangement according to claim 1, wherein the beam interchanging and image inverting system (89) comprises two shortened Porro prisms of the second kind (91c) which are inserted into one another to form a symmetric arrangement, the crown height of said two shortened Porro prisms of the second kind (91c) being smaller than that of a non-shortened Porro prism of the second kind (91).

4. Stereomicroscopy arrangement according to claim 2, wherein for a first viewer two first ocular arrangements are provided to which the beam bundles are supplied which are supplied to the objective arrangement (87) on the left-hand side (99, 137) and on the right-hand side (97, 161).

5. Stereomicroscopy system according to claim 4, further comprising a first magnification changing system (93) disposed in the beam path between the first ocular arrangements and the objective arrangement (87).

6. Stereomicroscopy system according to claim 4 or 5, wherein for a second viewer two further ocular arrangements are provided to which a beam bundle (133) which has been supplied to the objective arrangement (87) from above and a beam bundle (131) which has been supplied to the objective arrangement (87) from below are supplied.

7. Stereomicroscopy system according to claim 6, further comprising a second magnification changing system (93b) disposed between the further ocular arrangements and the objective arrangement (87).

8. Stereomicroscopy system according to claim 6 or 7, wherein one of the two Porro prisms of the second kind (153, 155) both supplies a beam bundle (157), directed downwards in the direction of the objective arrangement (87) from the object plane (117) in respect of a horizontal plane (95b) extending orthogonally to a central plane (135) of the objective arrangement (87), to the objective arrangement (87) as the beam bundle (133) supplied to the same from above, at the same time inverting image orientations, and supplies a beam bundle (159), directed upwards in the direction of the objective arrangement (87) from the object plane (117) in respect of the horizontal plane (95b), to the objective arrangement (87) as the beam bundle (131) supplied to the same from below, at the same time inverting image orientations.

9. Stereomicroscopy arrangement according to one of claims 1 to 8, wherein the beam interchanging and image inverting system (89) is pivotable or shiftable into the beam path.

10. Stereomicroscopy arrangement according to one of claims 1 to 9, further comprising an ophthalmoscopy lens system (115) for producing an intermediate image of an eye fundus (213) in the object plane (117) of the objective arrangement (87), wherein the ophthalmoscopy lens system (115)is removable from and re-insertable into the beam path together with the beam interchanging and image inverting system (89).

11. Stereomicroscopy system according to one of claims 1 to 9, further comprising an ophthalmoscopy lens system (115) for producing an intermediate image of an eye fundus (213) in the object plane (117) of the objective arrangement (87), wherein the beam interchanging and image inverting system (89) and the ophthalmoscopy lens system (115) are removable from the beam path in front of the objective arrangement (87), and wherein geometric dimensions and optical media of the beam interchanging and image inverting system (89) and focal lengths of the ophthalmoscopy lens system (115) are adjusted such that, when the beam interchanging and image inverting system (89) and the ophthalmoscopy lens system (115) are positioned in the beam path, the intermediate image of the eye fundus (213) is positionable in the object plane (117) of the objective arrangement (87), the objective arrangement (87) being spaced apart from the eye (211) by a distance b2 + b4, and, when the beam interchanging and image inverting system (89) and the ophthalmoscopic system are removed from the beam path, the cornea (121) of the eye (211) is positionable in the object plane (117) of the objective arrangement (87), the objective arrangement (87) being spaced apart from the eye (211) by a second distance b1, the distance b2+b4 and the distance b1 being substantially equal.

12. Stereomicroscopy system according to one of claims 1 to 11, wherein the difference between the distance b1 and the distance b2 is of from 15 mm to 40 mm, preferably, of from 20 mm to 30 mm and, particularly preferred, of from 24 mm to 26 mm.

## Revendications

1. Dispositif de microscopie stéréoscopique pour l'observation du fond d'oeil d'un oeil avec une lentille d'ophtalmoscopie dans un premier mode d'observation, et de la cornée d'un oeil dans un deuxième mode d'observation, comprenant :
un dispositif d'objectif (87) avec un plan objet (117) pour disposer l'objet ou l'image intermédiaire à observer, et un système de permutation de faisceau et de changement d'image (89),
**caractérisé en ce que** le système de permutation de faisceau et de changement d'image (89) dirige à droite un faisceau de rayonnement (101, 163) du dispositif d'objectif (87), dirigé depuis le plan objet (117) vers la gauche en direction du dispositif d'objectif (87), dirige à gauche un faisceau de rayonnement (97, 161) du dispositif d'objectif (87), dirigé depuis le plan objet (117) vers la droite en direction du dispositif d'objectif (87), et change chaque fois les orientations d'image des deux faisceaux de rayonnement (97, 101, 161, 163), où le système de permutation de faisceau et de changement d'image (89) peut être enlevé pour le deuxième mode d'observation, de la marche du rayon avant le dispositif d'objectif (87), où lorsque le système de permutation de faisceau et de changement d'image (89) est disposé dans la marche du rayon, le plan objet (117) du dispositif d'objectif (87) présente une distance b2 avec le dispositif d'objectif (87), où lorsque le système de permutation de faisceau et de changement d'image (89) est enlevé de la marche du rayon, le plan objet (117) présente une distance b1 avec le dispositif d'objectif (87), et où la différence entre la distance b1 et la distance b2 présente une valeur prédéterminée, telle que lors du changement du mode d'observation, essentiellement aucune modification de la distance entre le dispositif d'objectif (87) et l'oeil n'est nécessaire pour obtenir une image nette d'une part de la cornée, et d'autre part du fond d'oeil.

2. Dispositif de microscopie stéréoscopique selon la revendication 1, où le système de permutation de faisceau et de changement d'image (89) comprend au moins un prisme Porro de deuxième type (91, 153).

3. Dispositif de microscopie stéréoscopique selon la revendication 1, où le système de permutation de faisceau et de changement d'image (89) comprend deux prismes Porro de deuxième type (91c) raccourcis, enfilés en un agencement symétrique, où les deux prismes Porro de deuxième type (91c) raccourcis présentent chaque fois, un point culminant plus petit par rapport à un prisme Porro de deuxième type (91) non raccourci.

4. Dispositif de microscopie stéréoscopique selon la revendication 2, où deux premiers dispositifs oculaires sont prévus pour un premier observateur, qui sont traversés par les faisceaux de rayonnement entrant à gauche (99, 137) et à droite (97, 161) du dispositif d'objectif (87).

5. Dispositif de microscopie stéréoscopique selon la revendication 4, comprenant en outre, un premier système de modification - d'agrandissement (93), agencé dans la marche du rayon, entre les premiers dispositifs d'oculaire et le dispositif d'objectif (87).

6. Dispositif de microscopie stéréoscopique selon la revendication 4 ou 5, où deux autres dispositifs oculaires sont prévus pour un deuxième observateur, qui sont traversés pour l'un par un faisceau de rayonnement (133) entrant sous le dispositif d'objectif (87) et pour l'autre par un faisceau de rayonnement (131) entrant au-dessus du dispositif d'objectif (87).

7. Dispositif de microscopie stéréoscopique selon la revendication 6, comprenant en outre, un deuxième système de modification - d'agrandissement (93b), agencé entre les autres dispositifs oculaires et le dispositif d'objectif (87).

8. Dispositif de microscopie stéréoscopique selon la revendication 6 ou 7, où un des deux prismes Porro de deuxième type (153, 155) conduit non seulement, un des faisceaux de rayonnement (157) du dispositif d'objectif (87), dirigé depuis le plan objet (117) vers le bas en direction du dispositif d'objectif (87), par rapport à un plan horizontal (95b), perpendiculaire à un plan moyen (135) du dispositif d'objectif (87), avec changement de l'orientation de l'image, comme le faisceau de rayonnement (133) amené au-dessus de celui-ci, mais conduit également un des faisceaux de rayonnement (159) du dispositif d'objectif (87), dirigé depuis le plan objet (117) vers le haut en direction du dispositif d'objectif (87), par rapport au plan horizontal (95b), avec changement de l'orientation de l'image, comme le faisceau de rayonnement (131) amené au-dessous de celui-ci.

9. Dispositif de microscopie stéréoscopique selon l'une des revendications 1 à 8, où le système de permutation de faisceau et de changement d'image (89) peut être pivoté ou intercalé.

10. Dispositif de microscopie stéréoscopique selon l'une des revendications 1 à 9, comprenant en outre, un système de lentilles d'ophtalmoscopie (115) pour la production d'une image intermédiaire d'un fond d'oeil (213) dans le plan objet (117) du dispositif d'objectif (87), où le système de lentilles d'ophtalmoscopie (115) peut être enlevé et à nouveau, introduit dans la marche du rayon, simultanément au système de permutation de faisceau et de changement d'image (89).

11. Dispositif de microscopie stéréoscopique selon l'une des revendications 1 à 9, comprenant en outre, un système de lentilles d'ophtalmoscopie (115) pour la production d'une image intermédiaire d'un fond d'oeil (213) dans le plan objet (117) du dispositif d'objectif (87), où le système de permutation de faisceau et de changement d'image (89) et le système de lentilles d'ophtalmoscopie (115) peuvent être enlevés de la marche du rayon devant le dispositif d'objectif (87), et où les dimensions géométriques et le milieu optique du système de permutation de faisceau et de changement d'image (89) et les distances focales du système de lentilles d'ophtalmoscopie (115) sont déterminées de sorte que lorsque le système de permutation de faisceau et de changement d'image (89) et le système de lentilles d'ophtalmoscopie (115) sont agencés dans la marche du rayon, l'image intermédiaire du fond d'oeil (213) peut être aménagée dans le plan objet (117) du dispositif d'objectif (87) et le dispositif d'objectif (87) présente avec l'oeil (211), une distance b2+b4, et que lorsque le système de permutation de faisceau et de changement d'image (89) et le système de lentilles d'ophtalmoscopie (115) sont enlevés de la marche du rayon, la cornée (121) de l'oeil (211) peut être aménagée dans le plan objet (117) du dispositif d'objectif (87) et le dispositif d'objectif (87) présente avec l'oeil, une distance b1, et que la distance b2+b4 et la distance b1 sont essentiellement égales.

12. Dispositif de microscopie stéréoscopique selon l'une des revendications 1 à 11, où la différence entre la distance b1 et la distance b2, se situe dans la gamme allant de 15 mm à 40 mm, de préférence de 20 mm à 30 mm, et de manière plus préférée, de 24 mm à 26 mm.
